## Europäisches Patentamt
## European Patent Office
## Office européen des brevets

(19)

(11) Veröffentlichungsnummer: **0 114 922**

**A2**

(12) ## EUROPÄISCHE PATENTANMELDUNG

(21) Anmeldenummer: **83107064.4**

(22) Anmeldetag: **19.07.83**

(51) Int. Cl.³: **A 61 B 5/00**
**A 61 B 5/04, G 09 B 19/04**

(30) Priorität: **19.07.82 HU 234082**

(43) Veröffentlichungstag der Anmeldung:
**08.08.84 Patentblatt 84/32**

(84) Benannte Vertragsstaaten:
**AT BE CH DE FR GB IT LI LU NL SE**

(71) Anmelder: **KÖZPONTI VALTO-ES HITELBANK RT**
**INNOVACIOS ALAP**
**Szabadság tér 5**
**H-1054 Budapest(HU)**

(72) Erfinder: **Pataki, Laszlo, Dr.**
**Szabados u. 51**
**Budapest(HU)**

(72) Erfinder: **Gavajda, Peter**
**Jozsef u. 36**
**Budakeszi(HU)**

(72) Erfinder: **Patrovics, István**
**Martirok u. 26**
**Budapest(HU)**

(74) Vertreter: **Lehn, Werner, Dipl.-Ing. et al,**
**Hoffmann, Eitle & Partner Patentanwälte Arabellastrasse**
**4 (Sternhaus)**
**D-8000 München 81(DE)**

(54) **Einrichtung zur Wahrnehmung und Vermeidung hauptsächlich von Dysphonie-Symptomen im Kindesalter.**

(57) Die Erfindung bezieht sich auf eine Einrichtung hauptsächlich zur Wahrnehmung und Vermeidung von Dysphonie-Symptomen in Kindesalter.

Das gesteckté Ziel wird im Sinne der Erfindung durch eine Einrichtung erreicht, die dadurch charakterisiert werden kann, daß ein zweistufiger IC-Verstärker (3), der mit Meßfühlpunkten (2) verbunden ist und ein Potentiometer, das sich zwischen den einzelnen Stufen befindet, sowie einen symmetrischen Eingang besitzt, einerseits an einen LED-Indikator (4), der zweckmäßigerweise eine LED-Reihe (6) aus 16 Gliedern und einen IC Typ UAA 170, der in funktioneller Beziehung dazu steht, enthält, und andererseits an ein akustisches Warngerät (5), das einen mit einem Tonerteilungsgerät (7) zusammengebauten Gatterschaltkreis aus Kondensatoren und Widerständen sowie eine Zener-Diode, die mit letzterem verbunden ist, enthält, geschaltet ist.

Die Speiseeinheit (8) der Einrichtung enthält acht Baby-Zellen von je 1,5 V. Ihre maximale Stromaufnahme beträgt ca. 20 mA, so daß eine Garnitur Zellen mindestens 50 Betriebsstunden ermöglicht. Unter Berücksichtigung von Gesichtspunkten der Wirtschatlichkeit können auch die sog. Baby-Batterien verwendet werden. Ein Betrieb vom Netz aus ist nicht zweckmäßig, weil die Filterung der von dort kommenden Störungen und die Sicherung des Berührungsschutzes komplizierte und teure Schaltkreislösungen erforderlich machen würden. Nach dem Einschalten des Hauptschalters kann die die Zellenspannung an einem Indikator-Instrument, das direkt für diesen Zweck eingebaut wurd, kontrolliert werden.

Fig 1

EP 0 114 922 A2

Központi Valto- es Hitelbank Rt.


Einrichtung zur Wahrnehmung und Vermeidung hauptsächlich von Dysphonie-Symptomen im Kindesalter

---

Die Erfindung bezieht sich auf eine Einrichtung zur
Wahrnehmung und Vermeidung hauptsächlich von Dysphonie-Symptomen im Kindesalter.

Es ist bekannt, daß eine Tonerscheinung, die von der
normalen abweicht, eine primäre Erscheinung ist, die
eine organische Herkunft hat, oder funktional ist.
Die Funktionsstörung kann zu charakteristischen sekundären organischen Veränderungen führen.

Die Dysphonie, bei der die primäre organische Ursache
ausgeschlossen werden kann, ist als Erscheinung funktionaler Herkunft zu betrachten. Im engeren Sinne ist
unter dem Begriff Dysphonie eine funktionale Tonbildungsstörung zu verstehen, bei der das Tonbild nicht
nur vom Geräusch charakterisiert wird, sondern auch
die Pressung, die Spannung, der Tonbruch und eine plötzliche Änderung der Melodie oder auch die akustischen Attribute einer trägen, hypotonen Phonation ausgewiesen werden können. Dabei wird wohl die funktionale Dysphonie im Kindesalter von den obigen akustischen Merkmalen, u. zw. einem meist harten Tonbeginn während der Phonation, einer geschlossenen Mundstellung, einer Pressungs-Spannungs-Tontechnik, die sich auch auf die Hals- und Bauchmuskeln erstreckt, ätiologisch von der Psychogenität charakterisiert.

Ein Teil der Tonbildungsstörungen verschiedener Herkunft kann durch die Tonreedukation beseitigt werden.

Die schwierigste Aufgabe der Tonreedukation ist der weiche Tonbeginn und die Herausbildung einer pressungsfreien Tontechnik.

Die Halsmuskeln von Kindern, die an hyperkinetischer Dysphonie leiden, gelangen bei der Phonierung in einen gesteigerten Ton, sie werden gespannt und treten heraus. Durch ihre charakteristische Kopfhaltung - nach hinten und gleichzeitig nach oben - wird die Muskeltonsteigerung ebenfalls erhöht. Der gesteigerte Ton der Halsmuskeln kann durch verschiedene Meßverfahren ausgewiesen werden. Die elektrische Widerstandsänderungsmessung, das EMG, kann im Laboratorium angewandt werden und verursacht dem Kranken kleinere und größere Unannehmlichkeiten.

Ziel der Erfindung ist die Schaffung einer solchen Einrichtung zur Wahrnehmung und Vermeidung hauptsächlich von Dysphonie-Symptomen im Kindesalter, durch deren Anwendung die krankhafte Steigerung des Muskeltons auf solche Weise gut verfolgt werden kann, daß dies mit keinerlei physischen oder psychischen Unannehmlichkeiten für den Patienten im Kindesalter verbunden ist.

Ein weiteres Ziel besteht darin, daß die Einrichtung leicht bedient werden kann, der Patient ihren Fühler im gegebenen Fall selbst am eigenen Hals befestigt und auch der Kinderpatient anhand der Signale, die von der Einrichtung gegeben werden, bewußt oder automatisch die entsprechenden Konsequenzen ableitet.

Das gesteckte Ziel wird im Sinne der Erfindung durch eine Einrichtung erfüllt, die dadurch charakterisiert werden kann, daß ein zweistufiger IC-Verstärker, der mit Meßfühlpunkten verbunden ist und ein Potentiometer, das sich zwischen den einzelnen Stufen befindet, sowie einen symmetrischen Eingang besitzt, einerseits an einen LED-Indikator, der zweckmäßigerweise eine LED-Reihe aus 16 Gliedern und einen IC Typ UAA 170, der in funktioneller Beziehung dazu steht, enthält, und andererseits an ein akustisches Warngerät, das einen mit einem Tonerteilungsgerät zusammengebauten Gatterschaltkreis aus Kondensatoren und Widerständen sowie eine Zener-Diode, die mit letzterem verbunden ist, enthält, geschaltet ist.

Es hat sich gezeigt, daß die elektrische Spannung, die beim Anspannen der Halsmuskeln, die im Laufe der Phonation in einen gesteigerten Ton gelangen, an der Hautoberfläche entsteht, imstande ist, Signale, die proportional zum Ausmaß der Anspannung sind, zu induzieren, die zwischen der pressungsfreien Tonbildung und der Dysphonie im Augenblick der Bildung des Tones, der Verzerrung des Tones entsprechend, gemessen werden können.

Die krankhafte Muskeltonsteigerung kann mit der erfindungsgemäßen Einrichtung sehr gut verfolgt werden, ohne daß dem Kind auch nur die geringsten Unannehmlichkeiten verursacht werden. Durch eine entsprechende Kalibrierung kann erreicht werden, daß die Ausschläge die Differenzen, die sich aus der Tonstärke- bzw. Tonhöhenänderung ergeben, bei einer richtigen Sprechtechnik nicht anzeigen und das "Notsignal" nur bei einer krankhaften Muskelspannung gebildet wird.

Die Einrichtung selbst besteht aus einer Befestigungsarmatur, die Fühlungspole enthält, die auf der Hautoberfläche des Halses befestigt werden müssen, einem IC-Verstärker, der eng damit verbunden ist, und einem LED-Indikator, weiterhin aus einem akustischen Warngerät, das an eine Schwachstrom-Speiseeinheit angeschlossen ist.

Die Einrichtung gemäß der Erfindung ist im folgenden an einer vorteilhaften Ausführungsform und anhand der Zeichnungen näher erläutert. In den Zeichnungen zeigen

Fig. 1    eine Zusammenbau-Skizze der Einrichtung,

Fig. 2    eine Schaltskizze des IC-Verstärkers,

Fig. 3    eine Schaltskizze des LED-Indikators und

Fig. 4a und 4b eine Schaltskizze des akustischen
          Warngerätes

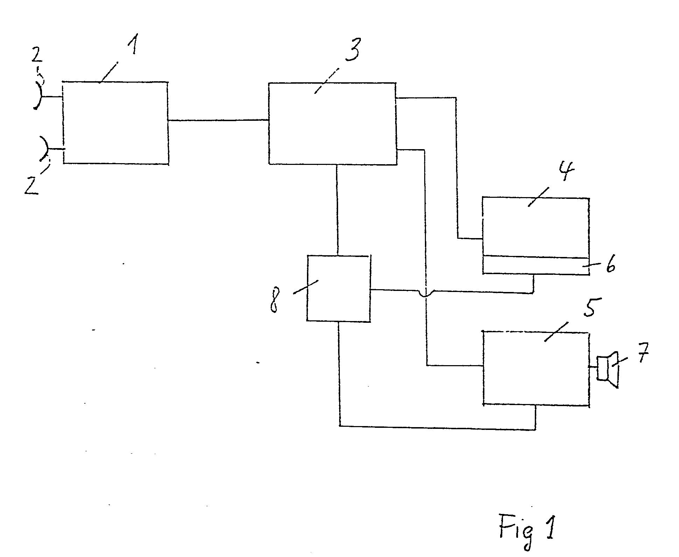

Aus Fig. 1 kann festgestellt werden, daß eine Befestigungsarmatur 1, die selbst eine elastische Ausbildung hat, Fühlungspole 2, die voneinander isoliert sind, enthält, von denen eine abgeschirmte elektrische Leitung zu einem IC-Verstärker 3 der Einrichtung führt.

Die Befestigungsarmatur 1 dient dazu, daß die Fühlungspole 2 an der entsprechenden Stelle des Halses des Patienten befestigt werden.

Der IC-Verstärker 3, der mit den Fühlrungspolen 2 in Verbindung steht, wird einerseits an einen LED-Indikator 4 und andererseits an ein akustisches Warngerät 5 angeschlossen. Der LED-Indikator 4 enthält eine LED-Reihe, die aus 16 Gliedern besteht, und ein wichtiges Konstruktionselement des akustischen Warngerätes 5 wird von einem Tonerteilungsgeärt 7 gebildet. Der IC-Verstärker 3, der LED-Indikator 4 und das akustische Warngeärt 5 sind mit einer Speiseeinheit 8 verbunden.

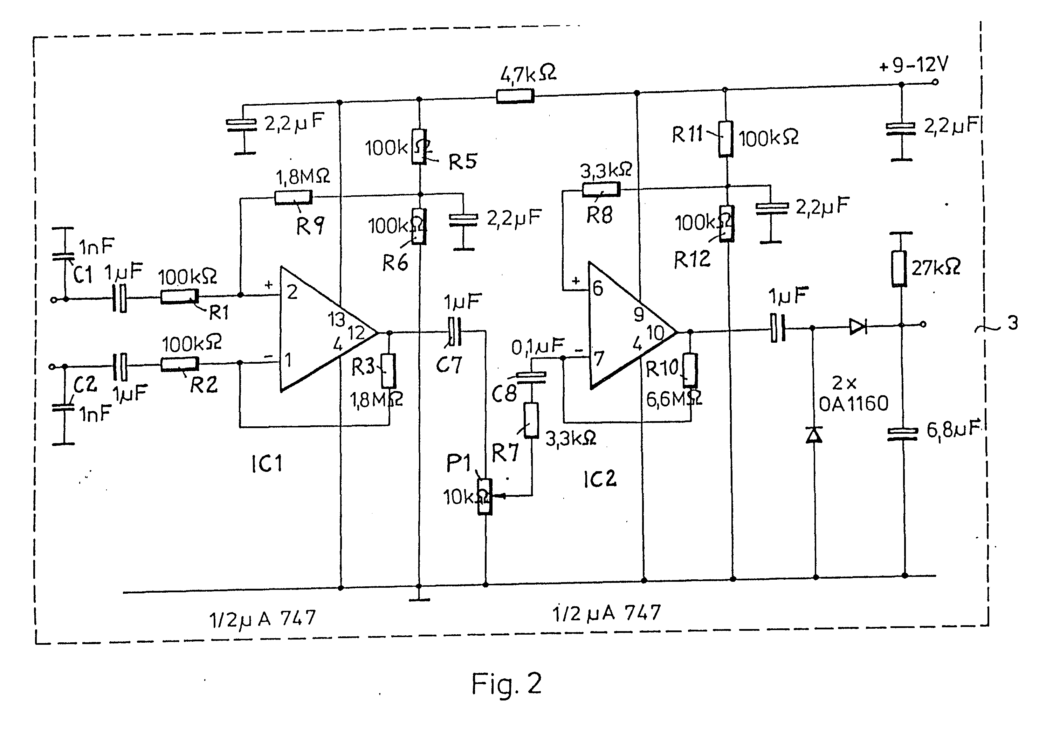

In Fig. 2 ist der IC-Verstärker dargestellt, wobei der Führungspol 2, der sich an der Muskulatur des Halses befindet, symbolisch dargestellt wurde.

Die elektrische Spannung, die beim Anspannen des fraglichen Muskels (der fraglichen Muskulatur) an der dazugehörigen Hautoberfläche entsteht, wird über ein zweiadriges abgeschirmtes Kabel in das Gerät geleitet. An dieser Stelle erfolgt die Filterung der Störungen, die sich aus der "Streuung" des Netzes ergeben, auf der üblichen Art und Weise mit Hilfe von Kondensatoren C 1 und C 2. Das Signal wird durch einen IC /uA 747, der zwei Verstärker /uA 741 in einem gemeinsamen Gehäuse, jedoch mit separater Speisespannungsversorgung enthält, verstärkt. Die einzelnen Stufen arbeiten als Differentialverstärker.

In Anbetracht dessen, daß eine sehr große Verstärkung erforderlich ist, wurde die erste Stufe (IC 1 = 1/2 /uA 747) auf eine kleinere und die zweite Stufe (IC 2 = 1/2 /uA 747) auf eine größere Verstärkung eingestellt. Dadurch kann erreicht werden, daß die Störungen nicht - oder zumindest nur in geringem Maße - die nützlichen Signale gelangen. Die richtige Einstellung des IC 1 erfolgt mit Hilfe von Widerständen R 9 und R 3. Im Interesse der Sicherung des symmetrischen Eingangs werden die Elemente C 1 und C 2 sowie R 1 und R 2 nach einem sog. Zusammensuchen eingebaut (C 1 = C 2; R 1 = R 2).

Der nicht invertierende Eingang liegt an einem "O"-Potential, was mit Hilfe des Teilers, der aus Widerständen R 5 und R 6 besteht, erfolgt (R 5 = R 6).

Von einem Kondensator C 6 werden Wechselstromstörungen in die Erde geleitet. Der Wert eines Rückkopplungswiderstandes R 3 darf nicht größer als 3 MOhm sein, weil dadurch sonst eine derart große Verstärkung erzeugt würde, die zur Erregung des Systems führen würde. Der IC 2 arbeitet in einer ähnlichen Schaltung, wobei lediglich die zusätzlichen Bestandteile derart dimensioniert sind, daß seine Verstärkung bedeutend größer ist, als die der ersten Stufe.

Zwischen den beiden Stufen befindet sich ein "Empfindlichkeitsregel-Potentiometer" P 1, das zwischen den Ausgang des IC 1 und die "Erde" geschaltet ist. In seiner "unteren" Endstellung gelangt der Invertiereingang des IC 2 über einen Widerstand R 7 an die "Erde" und sein nicht invertierender Eingang über einen Widerstand R 8 an das "0"-Potential, das von einem Teiler, der aus Widerständen R 11 und R 12 besteht, erzeugt wird, das heißt am Ausgang des IC 2 liegt keine Spannung an. Wenn der Gleitschieber des Potentiometers P 1 in die "obere" Endstellung geschoben wird, gelangt das vom IC 1 verstärkte Signal über Kopplungskondensatoren C 7 und C 8 sowie den Widerstand R 7 an den Invertiereingang des IC 2.

Der Wert eines Rückkopplungswiderstandes R 10 liegt bei der beschriebenen Ausführungsform zwischen 5 MOhm und 10 MOhm. Auch bei dieser Stufe muß darauf geachtet werden, daß die Glieder (R 11 und R 12) des Teilers, der das "0"-Potential erzeugt, genau gleich sind. Der Ausgang des IC 2 wird nach einer Zweiwege-Gleichrichtung an die weiteren Stufen ausgeführt.

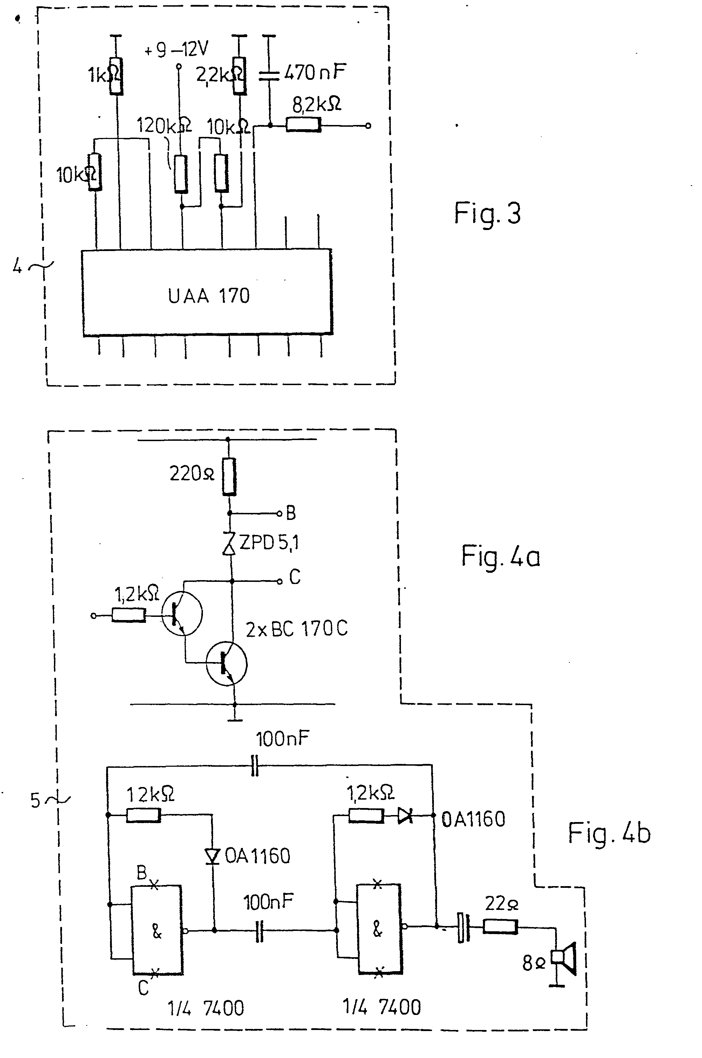

Der LED-Indikator, der in Fig. 3 gezeigt ist, besteht aus einer LED-Reihe 6 aus 16 Gliedern. Diese LED-Reihe wird von einem IC Tpy UAA 170 angetrieben.

Der Eingangspunkt ist mit Hilfe eines Kondensators von 470 nF zur Vibrierungsverminderung der Leuchtdioden mit der Erde verbunden. Das Signal würde nämlich aufgrund seines Charakters ein Herunter- und heraufspringen des Leuchtstreifens, der an der LED-Reihe erscheint, um mehrere Grad verursachen, wodurch der Prozeß der Tonreedukation gestört würde. Die LED-Reihe hat eine Empfindlichkeit von ca. 0 - 0,5 V.

In den Fig. 4a und 4b ist eine Schaltskizze des akustischen Warngerätes gezeigt.

Das akustische Warngerät 5 überträgt das Warnsignal über ein Tonerteilungsgerät 7, vor das ein symmetrischer Gatterschaltkreis, der Kondensatoren von 100 nF und auch Widerstände von 1,2 kOhm enthält, geschaltet ist. Dieser Schaltkreis ist an seinen D- und C-Stellen an eine Zener-Diode Z angeschlossen. Wenn die induzierte Spannung größer als die Spannung ist, die von der LED-Skala umfaßt werden kann, wird von einem Warnton gemeldet, daß das Potentiometer 9 der Verstärkungsregelung auf eine niedrigere Teilung gestellt werden muß. Diese Einheit enthält einen astabilen Multivibrator, dessen Speisespannung von einer Zener-Diode von 5,1 V, die sich im Kollektorkreis eines Transistorpaares in Darlington-Schaltung befindet, eingestellt wird.

Eine Speiseeinheit 8 der Einrichtung enthält acht Baby-Zellen von je 1,5 V. Ihre maximale Stromaufnahme beträgt ca. 20 mA, so daß eine Garnitur Zellen mindestens 50 Betriebsstunden ermöglicht. Unter Berücksichtigung von Gesichtspunkten der Wirtschaftlichkeit können auch die sog. Baby-Batrerien verwendet werden. Ein Betrieb vom netz aus ist nicht zweckmäßig, weil die Filterung der von dort kommenden Störungen und die Sicherung des Berührungsschutzes komplizierte und teure Schaltkreislösungen erforderlich machen würden. Nach dem Einschalten des Hauptschalters kann die Zellenspannung an einem Indikator-Instrument, das direkt für diesen Zweck eingebaut wurde, kontrolliert werden.

Patentansprüche:

1.    Einrichtung zur Wahrnehmung und Vermeidung hauptsächlich von Dysphonie-Symptomen im Kindesalter, aus Fühlungspolen (2), die an einen IC-Verstärker (3) angeschlossen sind, einem LED-Indikator (4) und einem akustischen Warngerät (5) als Hauptkonstruktionselemente zusammengebaut, dadurch g e k e n n - z e i c h n e t , daß ein zweistufiger IC-Verstärker (3), der ein Potentiometer (P1), das sich zwischen den einzelnen Stufen befindet, sowie einen symmetrischen Eingang besitzt, einersetis an einen LED-Indikator (4), der zweckmäßigerweise eine LED-Reihe (6) aus sechzehn Gliedern und einen IC Typ UAA 170, der in funktioneller Beziehung dazu steht, enthält, und andererseits an ein akustisches Warngerät (5), das einen mit einem Tonerteilungsgerät (7) zusammengebauten Gatterschaltkreis aus Kondensatoren 100 nF und Widerständen 1,2 kOhm sowie eine Zener-Diode Z, die mit letzterem verbunden ist, enthält, über voneinander getrennte Leitungen an eine Speiseeinheit (8) angeschlossen ist.

2.    Einrichtung nach Anspruch 1, dadurch g e - k e n n z e i c h n e t , daß die erste Stufe (IC1 = 1/2 uA 747) des IC-Verstärkers (3) auf eine kleinere und die zweite Stufe (IC2 = 1/2 uA 747) auf eine größere Verstärkung eingestellt ist.

3.    Einrichtung nach Anspruch 1 oder 2, dadurch g e k e n n z e i c h n e t , daß der Eingangspunkt des LED-Indikators (4) mittels eines Kondensators von 470 nF mit der Erde verbunden ist.

Fig 1

Fig. 2

Fig. 3

Fig. 4a

Fig. 4b